# EUROPÄISCHE PATENTSCHRIFT

(11) **EP 1 797 920 B1**
(45) Veröffentlichungstag und Bekanntmachung des Hinweises auf die Patenterteilung: **19.10.2011**
(21) Anmeldenummer: 06001463.6
(22) Anmeldetag: 24.01.2006
(51) Int. Cl.: A61M 5/178, A61M 5/31

(54) **Vorrichtung zur Aufnahme und zum Austragen einer fliessfähigen Substanz**
Device suitable for retaining and delivering a flowable substance
Dispositif apte à recevoir et à délivrer une substance fluide

(30) Priorität: 15.12.2005 EP 05027547
(43) Veröffentlichungstag der Anmeldung: 20.06.2007
(73) Patentinhaber: Sulzer Mixpac AG, 9469 Haag (CH)
(72) Erfinder: Sogaro, Alberto, 61476 Kronberg (DE)
(74) Vertreter: advotec.

(56) Entgegenhaltungen:
- EP-A- 0 112 574
- WO-A-00/20057
- WO-A-96/18552
- WO-A-03/100424
- US-A- 3 157 323
- US-A- 5 135 507

## Beschreibung

Die Erfindung betrifft eine Vorrichtung zur Aufnahme und zum Austragen einer fließfähigen Substanz mit den Merkmalen des Oberbegriffs des Patentanspruchs 1.

Eine derartige Vorrichtung ist aus Praxis bekannt, sowie im Dokument EP-A-0 112 574 offenbart, und wird beispielsweise im medizinischen Bereich in Form einer Spritze zum Injizieren von Medikamenten eingesetzt.

Eine derartige Spritze umfasst einen Aufnahmekörper, in dem ein Aufnahmeraum für die fließfähige Substanz ausgebildet ist und in dem ein Kolben axial verschieblich geführt ist. An seiner dem Kolben abgewandten Stirnseite ist der Aufnahmekörper mit einem im Wesentlichen zylindrischen Austragsstutzen versehen, auf dem eine Kanüle oder auch eine andere Applikationseinrichtung aufgesetzt werden kann. Derartige Spritzen sind in der Regel als Einwegspritzen ausgeführt und werden nach ihrem Gebrauch entsorgt.

Der Erfindung liegt die Aufgabe zugrunde, eine gemäß der einleitenden genannten Gattung ausgebildete Vorrichtung bereitzustellen, die für Mehrfachanwendungen geeignet ist, wobei die fließfähige Substanz zwischen den einzelnen Anwendungen geschützt in dem Aufnahmeraum des Aufnahmekörpers vorgehalten werden kann.

Diese Aufgabe ist erfindungsgemäß durch die Vorrichtung mit den Merkmalen des Patentanspruchs 1 gelöst.

Erfindungsgemäß ist also an der Innenseite des Austragsstutzens ein Verschlusszapfen geführt, der von mindestens einem Querkanal durchsetzt ist, von dem ein Axialkanal abzweigt. In einer Schließstellung des Verschlußzapfens ist eine Fließverbindung zwischen dem Querkanal und dem Aufnahmeraum gesperrt. In einer Aktivierungsstellung des Verschlusszapfens ist eine Fließverbindung zwischen dem Querkanal und dem Aufnahmeraum freigegeben, so dass die fließfähige Substanz über den Querkanal und den Axialkanal aus dem Aufnahmeraum austragbar ist.

Erfindungsgemäß wird also eine Vorrichtung zur Aufnahme und zum Austragen einer fließfähigen Substanz bereitgestellt, die nach Art einer Spritze ausgebildet ist. Der Verschlusszapfen ist nach Art eines Stopfens ausgebildet, der in den Austragsstutzen eingesetzt ist und dessen Positionierung in dem Auslassstutzen die Ausgabe der fließfähigen Substanz entweder sperrt oder freigibt.

Der Aufnahmekörper und damit der an den Aufnahmekörper angeformte Austragsstutzen sind vorzugsweise aus einem chemisch beständigen Kunststoff gefertigt, so dass sich die Vorrichtung insbesondere auch in Verbindung mit Medikamenten und/oder Prüfsubstanzen einsetzen lässt, die im Bereich der Analytik verwendet werden.

Um einen zusätzlichen Stoff in den Aufnahmeraum einbringen und dort in einer Prüfflüssigkeit lösen zu können, weist eine spezielle Ausführungsform der Vorrichtung nach Erfindung an dem Aufnahmekörper einen seitlichen Einführkanal für den Stoff auf. Beispielsweise wird über den Einführkanal ein mit einer Probe beaufschlagtes Wattestäbchen in eine bereits in dem Aufnahmeraum enthaltende Prüfflüssigkeit eingebracht, so dass die Probe in der Prüfflüssigkeit gelöst wird. Die mit der Probe beaufschlagte Flüssigkeit kann dann nach dem Verbringen des Verschlusszapfens in Aktivierungsstellung zu Analysezwecken durch Betätigung des Kolbens aus der Vorrichtung ausgetragen werden. Durch den seitlichen Einführkanal kann auch bei zurückgezogenem Kolben Luft in einen oberhalb der Prüfflüssigkeit angeordneten Raum strömen, so dass diese beim Drücken des Kolbens zumindest anteilig in der Prüfflüssigkeit in Lösung gebracht werden kann.

Der Einführkanal kann insbesondere als einseitige trichterartige Aufweitung einer Seitenwand des Aufnahmekörpers in dem dem Austragsstutzen abgewandten Endbereich sein.

Bei einer speziellen Ausführungsform der Vorrichtung nach der Erfindung weist der Austragstutzen an seiner Innenseite mindestens eine ringförmige Dichtlippe auf, an der der Verschlusszapfen geführt ist. Eine derartig ausgelegte Vorrichtung arbeitet derart, dass der Querkanal in einer Schließstellung des Verschlusszapfens auf der dem Aufnahmeraum abgewandten Seite der Dichtlippe angeordnet ist und in einer Aktivierungsstellung des Verschlusszapfens mit dem Aufnahmeraum in Verbindung steht.

Bevorzugt sind an der Innenseite des Austragstutzens zwei ringförmige Dichtlippen vorgesehen und hat der Querkanal einen Durchmesser, der gleich dem oder kleiner als der Abstand zwischen den beiden Dichtlippen ist.

Bei einer alternativen Ausführungsform der Vorrichtung nach der Erfindung greift der Verschlusszapfen zum Sperren der Fließverbindung zwischen dem Aufnahmeraum und dem Querkanal in eine Auslassöffnung des Aufnahmeraums dichtend ein. Bei einer derartig ausgelegten Vorrichtung hat der Verschlusszapfen zur Erhöhung der Dichtwirkung in Schließstellung vorzugsweise eine konische Dichtfläche, die mit einer korrespondierenden Fläche des Austragstutzens zusammenwirkt, die sich an die Auslassöffnung anschließt.

Der Verschlusszapfen, der mit den Dichtlippen an der Innenseite des Austragsstutzens zusammenwirkt, kann insbesondere Bestandteil einer Applikationseinrichtung, wie einer Kanüle, einer Pipettierspitze oder dergleichen sein. Denkbar ist es aber auch, den Verschlusszapfen als getrenntes Bauteil auszubilden, der beim Aufsetzen einer Applikationseinrichtung auf den Austragsstutzen in Richtung des Aufnahmeraums des Aufnahmekörpers gedrückt und in Aktivierungsstellung verschoben wird, so dass ein Strömungsweg zwischen dem Aufnahmeraum und der Applikationseinrichtung über den Querkanal und den Axialkanal des Verschlusszapfens hergestellt wird.

Die Applikationseinrichtung kann auch mit einer Düse versehen sein, über die bei Betätigung des Kolbens die in dem Aufnahmeraum enthaltene Substanz nach Verbringen des Verschlusszapfens in Aktivierungsstellung beispielsweise in zerstäubter Form ausgebracht werden kann.

Um eine selbsttätig wiederverschließbare Vorrichtung zu realisieren, kann der Verschlusszapfen in Schließrichtung vorgespannt sein. So wird der Verschlusszapfen beispielsweise beim Aufsetzen einer Applikationseinrichtung in Aktivierungsstellung verschoben, die beim Abnehmen der Applikationseinrichtung wieder aufgehoben wird, so dass der Verschlusszapfen selbsttätig in Schließstellung zurückschnappt.

Damit die Feder nicht mit der fließfähigen Substanz in Berührung kommt und diese gegebenenfalls kontaminiert, ist die Feder bei einer speziellen Ausführungsform der Vorrichtung nach der Erfindung gekapselt gelagert.

Beispielsweise umgreift die Feder den Austragsstutzen, wobei sie sich einerseits an einem Auflager des Austragstutzens und andererseits an dem Verschlusszapfen oder einer fest mit dem Verschlusszapfen verbundenen Applikationseinrichtung abstützt.

Um zu verhindern, dass der Verschlusszapfen sich beim Betätigen des Kolbens von dem Austragsstutzen löst, ist der Verschlusszapfen vorzugsweise an dem Austragsstutzen verrastet.

Bei einer speziellen Ausführungsform der Vorrichtung nach der Erfindung weist eine Applikatinseinrichtung oder Adaptereinrichtung, an der der Verschlusszapfen ausgebildet ist, mindestens zwei Schenkel auf, die von einer Grundplatte in Richtung des Aufnahmeraums im Wesentlichen parallel zur Achse des Axialkanals vorspringen und an denen jeweils eine Rastnase ausgebildet ist, die mit einem korrespondierenden Rastbund am Umfang des Austragsstutzens zusammenwirkt. Eine derartige Rasteinrichtung gewährleistet, dass die Applikationseinrichtung bzw. Adaptereinrichtung mit dem Verschlusszapfen sich beim Ausbringen der fließfähigen Substanz aus dem Aufnahmeraum nicht von dem Austragstutzen löst.

Vorzugsweise sind an der Außenseite des Austragsstutzens Fixiereinrichtungen ausgebildet, die mit korrespondierenden Bauelementen des Verschlusszapfens zusammenwirken und so diesen in Schließstellung und/oder in Aktivierungsstellung sichern können.

Derartige Fixiereinrichtungen und auch Rasteinrichtungen an der Außenseite des Austragsstutzens können insbesondere auch als Auflager für die Feder zur in Schließstellung vorgespannten Lagerung des Verschlusszapfens dienen.

Weitere Vorteile und vorteilhafte Ausgestaltungen des Gegenstands nach der Erfindung sind der Beschreibung, der Zeichnung und den Patentansprüchen entnehmbar.

Drei Ausführungsbeispiele einer erfindungsgemäß ausgelegten Vorrichtung zur Aufnahme und zum Austragen einer fließfähigen Substanz sind in der Zeichnung schematisch vereinfacht dargestellt und werden in der nachfolgenden Beschreibung näher erläutert. Es zeigt:
- Fig. 1: eine Seitenansicht einer spritzenartigen Vorrichtung in Schließstellung;
- Fig. 2: die Vorrichtung nach Fig. 1 mit zurückgezogenem Kolben:
- Fig. 3: die Vorrichtung nach Fig. 1 in Aktivierungsstellung;
- Fig. 4: die Vorrichtung nach Fig. 1 in Aktivierungsstellung nach der Entleerung;
- Fig. 5: einen Längsschnitt durch einen Aufnahmekörper der Vorrichtung nach Fig. 1;
- Fig. 6: eine Stirnansicht des Aufnahmekörpers;
- Fig. 7: einen Schnitt durch eine erfindungsgemäß ausgebildete Vorrichtung mit einer selbsttätigen Verschlusseinrichtung im Bereich eines Austragstutzens;
- Fig. 8: einen Längsschnitt durch einen weitere Ausführungsform in Schließstellung;
- Fig. 9: einen Längsschnitt durch die Vorrichtung nach Fig. 8 im aktivierten Zustand;
- Fig. 10: einen Längsschnitt durch die Vorrichtung nach Fig. 9 im entleerten Zustand;
- Fig. 11: einen Längsschnitt durch einen Aufnahmekörper der Vorrichtung nach Fig. 8 in Alleinstellung;
- Fig. 12: eine Stirnansicht des Aufnahmekörpers;
- Fig. 13: einen Längsschnitt durch eine Adaptereinrichtung der Vorrichtung nach Fig. 8;
- Fig. 14: eine Stirnansicht der Adaptereinrichtung;
- Fig. 15: einen Längsschnitt durch eine dritte Ausführungsform einer erfindungsgemäß ausgebildeten, spritzenartigen Vorrichtung in Schließstellung;
- Fig. 16: die spritzenartige Vorrichtung nach Fig. 15 in Aktivierungsstellung;
- Fig. 17: die spritzenartige Vorrichtung nach Fig. 15 in entleertem Zustand;
- Fig. 18: einen Längsschnitt durch eine Applikationseinheit der spritzenartigen Vorrichtung nach den Figuren 15 bis 17; und
- Fig. 19: eine Seitenansicht der Applikationseinheit nach Fig. 18.

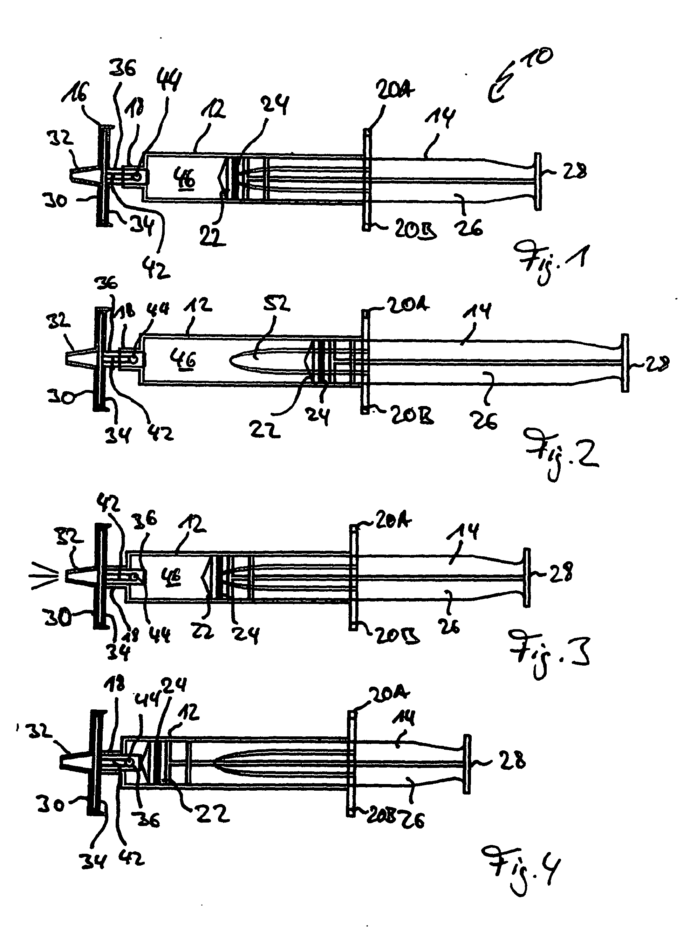

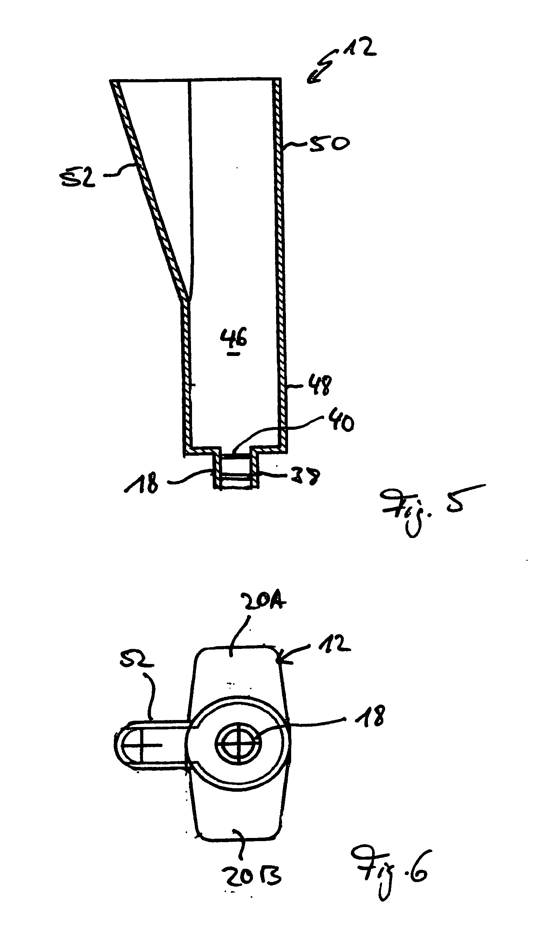

In den Figuren 1 bis 6 ist eine Vorrichtung 10 zum Aufbewahren und Austragen einer fließfähigen Substanz, beispielsweise einer mit einer Speichelprobe beaufschlagten Prüfflüssigkeit dargestellt. Die Vorrichtung 10 umfasst im Wesentlichen drei Baueinheiten, nämlich einen im Wesentlichen zylindrischen Aufnahmekörper 12, eine Kolbeneinheit 14 und eine Applikationseinheit 16.

Der Aufnahmekörper 12 umfasst einen im Wesentlichen zylindrischen Grundkörper, der an einer Stirnseite mit einem Austragsstutzen 18 versehen ist, der ebenfalls zylindrisch ausgebildet ist.

An seiner dem Austragsstutzen 18 abgewandten Stirnseite weist der Aufnahmekörper 12 zwei Greiflaschen 20A und 20B auf, an denen bei Betätigung der Kolbeneinheit 14 beispielsweise ein Ring- und ein Mittelfinger eines Benutzers anliegen können.

Die Kolbeneinheit 14 weist einen zylindrischen Kolben 22 auf, der an seinem Umfang mit einer Dichtlippe 24 versehen ist. Der Kolben 22 ist über eine Kolbenstange 26 mit einer Drückplatte 28 zur Anlage eines Fingers, insbesondere eines Daumens des Benutzers verbunden.

Die Applikationseinheit 16 ist als Applikationseinrichtung ausgebildet, die aus einer Stirnplatte 30 mit einer Düse 32 und einer Verschlussplatte 34 gebildet ist, an der ein Verschlusszapfen 36 angeformt ist. Zwischen der stirnseitigen Platte 30 und der Verschlussplatte 34 kann ein hier nicht näher dargestelltes Filter oder dergleichen angeordnet sein.

Der Verschlusszapfen 36 ist in den Austragsstutzen 18 des Aufnahmekörpers 12 eingesetzt und wirkt mit zwei in axialer Richtung des Aufnahmekörpers 12 voneinander beabstandeten Dichtlippen 38 und 40 zusammen, die an der Innenseite des Austragsstutzens 18 einstückig ausgebildet sind.

Der Verschlusszapfen 36 weist einen Axialkanal 42 auf, der an der der Stirnseitenplatte 30 zugewandten Seite der Verschlussplatte 34 mündet und mit einem Querkanal 44 verbunden ist, der den Verschlusszapfen 36 durchgreift. Der Querkanal 44 hat einen Durchmesser, der etwas geringer ist als der axiale Abstand zwischen den beiden Dichtlippen 38 und 40, so dass der Verschlusszapfen 36 in einer Schließstellung angeordnet ist, wenn der Querkanal 44 zwischen den beiden Dichtlippen 38 und 40 angeordnet ist, und in einer Aktivierungsstellung angeordnet ist, wenn der Querkanal 44 über die Dichtlippen 40 hinweg in einen Aufnahmeraum 46 des Aufnahmekörpers 12 verschoben ist, so dass eine in dem Aufnahmeraum 46 enthaltene Flüssigkeit durch Drücken der Kolbeneinheit 14 über den Querkanal 44, den Axialkanal 42 und die Düse 32 ausgetragen werden kann.

Des Weiteren umfasst der Aufnahmekörper 12 einen vorderen zylindrischen Abschnitt 48, an welchen sich ein unterbrochen zylindrischer Abschnitt 50 anschließt, der mit einem sich trichterartig nach außen öffnenden Einführkanal 52 versehen ist. Die Benutzung der Vorrichtung 10 wird nachfolgend an Hand der Figuren 1 bis 4 näher erläutert.

In einer Ausgangsstellung befindet sich der Verschlusszapfen 36 die Applikationseinheit 16 in einer Schließstellung, in der der Querkanal 44 zwischen den beiden Dichtlippen 38 und 40 angeordnet ist. Des Weiteren ist der Aufnahmeraum 46 mit einer Prüfflüssigkeit befüllt und ist der Kolben 22 so weit in Richtung des Austragsstutzens 18 in den Aufnahmekörper 12 eingeschoben, dass er den Aufnahmeraum 46 in Richtung des Einführkanals 52 dichtet. Der Kolben 22 liegt also in dem vorderen zylindrischen Abschnitt 48 des Aufnahmekörpers 12.

In einem zweiten Schritt wird der Kolben 22 in die in Fig. 2 dargestellte Stellung zurückgezogen, so dass über den Einführkanal 52 ein mit einer Speichelprobe beaufschlagtes Wattestäbchen oder dergleichen in die in dem Aufnahmeraum 46 enthaltene Flüssigkeit eingetaucht werden kann. Anschließend wird das Wattestäbchen wieder über den Einführkanal 52 aus dem Aufnahmekörper 12 herausgezogen.

In einem dritten Schritt wird nun der Kolben 22 wieder in die in Fig. 1 dargestellte Stellung verfahren.

In einem nächsten, vierten Schritt wird die Applikationseinheit 16 in Richtung des Aufnahmekörpers 12 verschoben, so dass sich der Verschlusszapfen 36 in einer Aktivierungsstellung befindet und ein Strömungspfad zwischen dem Aufnahmeraum 46 und der Düse 32 über den Querkanal 44 und den Axialkanal 42 hergestellt ist. Durch einen Druck auf die Drückplatte 28 der Kolbeneinheit 14 kann nun die mit der Speichelprobe beaufschlagte Prüfflüssigkeit über die Düse 32 aus dem Aufnahmekörper 12 ausgetragen werden.

In einer vorderen Endstellung, in der der Aufnahmeraum 46 im Wesentlichen entleert ist, liegt dann die Stirnseite des Kolbens 22 an der freien Stirnseite des Verschlusszapfens 36 der Applikationseinheit 16 an.

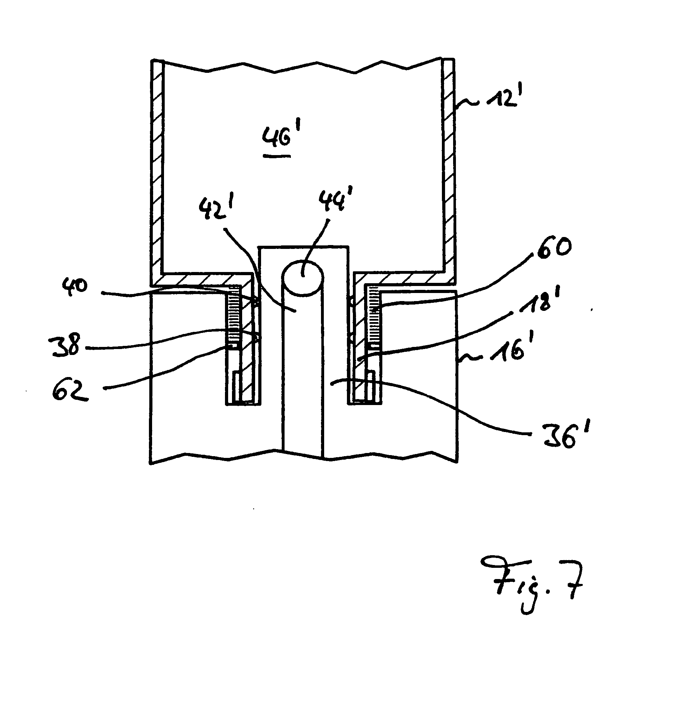

In Fig. 7 ist ein vorderer Bereich eines Aufnahmekörper 12' der in den Figuren 1 bis 6 gezeigten Art dargestellt, auf den eine Applikationseinrichtung 16' aufgesetzt ist, die beispielsweise eine Pipettierspitze, eine Kanüle oder dergleichen sein kann.

Die Applikationseinrichtung 16' ist mit einem Verschlusszapfen 36' der in den Figuren 1 bis 4 näher dargestellten Art versehen, der mithin in einen Austragstutzen 18' des Aufnahmekörpers 12' eingreift und einen Querkanal 44' und einen Axialkanal 42' umfasst, wobei der Querkanal 44' mit Dichtlippen 38 und 40 zusammenwirkt, die an der Innenseite des Austragsstutzens 18' des Aufnahmekörpers 12' ausgebildet sind, auf den die Applikationseinrichtung 16' aufgesetzt ist.

Die Applikationseinrichtung 16' und damit auch der Verschlusszapfen 36' sind mittels einer den Austragsstutzen 18' umgreifenden Spiralfeder 60 in Schließstellung des Verschlusszapfens 36 vorgespannt, so dass der Verschlusszapfen 36' bei entsprechender Freigabe selbsttätig aus der in Fig. 7 dargestellten Aktivierungsstellung in Schließstellung zürückschnappt.

Die Spiralfeder 60 ist hierzu zwischen einer ein Auflager bildenden Stirnfläche des Aufnahmekörpers 12' und einem Ringbund 62 der Applikationseinrichtung 16' eingespannt.

Damit sich die Applikationseinrichtung 16' nicht versehentlich von dem Aufnahmekörper 12' lösen kann, sind vorzugsweise zwischen der Applikationseinrichtung 16' und dem Austragsstutzen 18' Rastmittel angeordnet, die aus einem Ringbund 64 des Austragstutzens 36' gebildet sind. Diese Rastmittel können auch als Fixiermittel zur Festlegung der Schließstellung und der Aktivierungsstellung des Verschlusszapfens 36' darstellen.

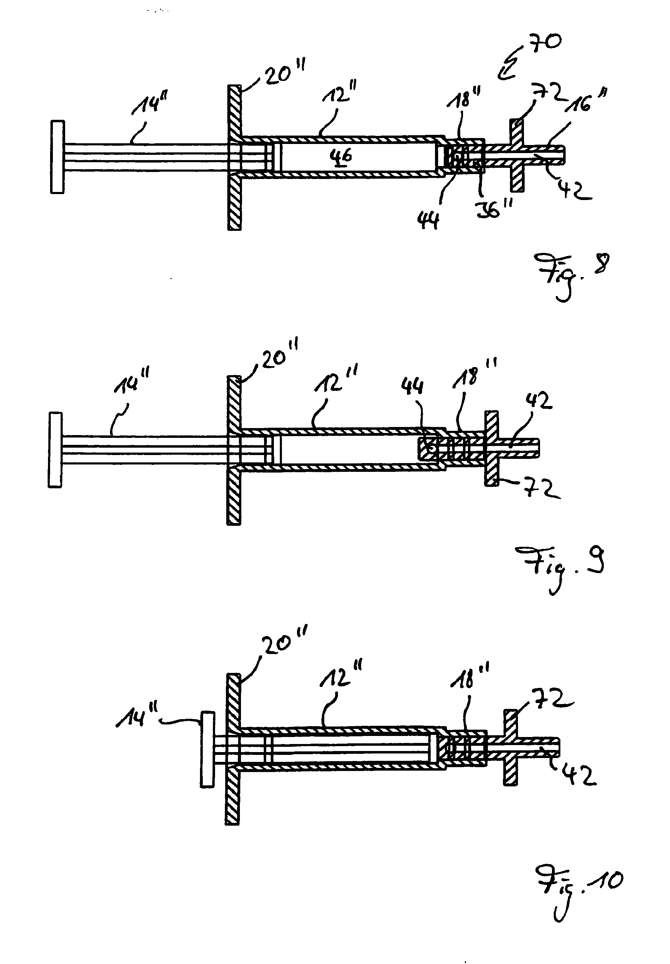

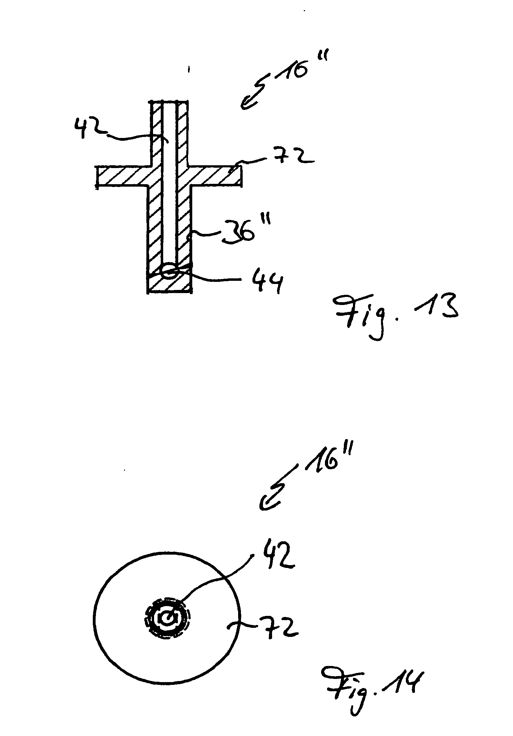

In den Figuren 8 bis 14 ist eine weitere Ausführungsform einer erfindungsgemäß ausgebildeten Vorrichtung 70 zum Aufbewahren und Austragen einer fließfähigen Substanz dargestellt. Die Vorrichtung 10 umfasst wiederum im Wesentlichen drei Baueinheiten, nämlich einen im Wesentlichen zylindrischen Aufnahmekörper 12", eine Kolbeneinheit 14" und eine Adaptereinrichtung 16".

Der Aufnahmekörper 12" umfasst einen im Wesentlichen zylindrischen Grundkörper, der an einer Stirnseite mit einem Austragsstutzen 18" versehen ist, der ebenfalls zylindrisch ausgebildet ist.

An seiner dem Austragsstutzen 18" abgewandten Stirnseite weist der Aufnahmekörper 12" elliptischen Bund 20" auf, an dem bei Betätigung der Kolbeneinheit 14" beispielsweise ein Ring- und ein Mittelfinger eines Benutzers anliegen können.

Die Kolbeneinheit 14" ist entsprechend der Kolbeneinheit der Ausführungsform nach den Figuren 1 bis 6 ausgebildet.

Die Adaptereinrichtung 16" ist aus einem Rohrstück gebildet, dessen dem Aufnahmekörper 12" zugewandtes Ende einen Verschlusszapfen 36" bildet und dessen dem Aufnahmekörper 12" abgewandtes Ende zum Verbinden mit einem Applikator, wie einer Kanüle, oder einem Schlauch ausgebildet ist.

Der Verschlusszapfen 36'' ist in den Austragsstutzen 18" des Aufnahmekörpers 12" eingesetzt und wirkt mit zwei in axialer Richtung des Aufnahmekörpers 12" voneinander beabstandeten Dichtlippen 38 und 40 zusammen, die an der Innenseite des Austragsstutzens 18 einstückig ausgebildet sind.

Der Verschlusszapfen 36" weist einen Axialkanal 42 auf, der an der der Stirnseitenplatte 30 zugewandten Seite der Verschlussplatte 34 mündet und mit einem Querkanal 44 verbunden ist, der den Verschlusszapfen 36" durchgreift. Der Querkanal 44 hat einen Durchmesser, der etwas geringer ist als der axiale Abstand zwischen den beiden Dichtlippen 38 und 40, so dass der Verschlusszapfen 36" in einer Schließstellung angeordnet ist, wenn der Querkanal 44 zwischen den beiden Dichtlippen 38 und 40 angeordnet ist, und in einer Aktivierungsstellung angeordnet ist, wenn der Querkanal 44 über die Dichtlippen 40 hinweg in einen Aufnahmeraum 46 des Aufnahmekörpers 12" verschoben ist, so dass eine in dem Aufnahmeraum 46 enthaltene Flüssigkeit durch Drücken der Kolbeneinheit 14" über den Querkanal 44, den Axialkanal 42 ausgetragen werden kann.

In Fig. 8 ist die Vorrichtung 70 im deaktivierten Zustand dargestellt. In Fig. 9 ist die Vorrichtung 70 im aktivierten Zustand dargestellt, in dem die Adaptereinrichtung 16" soweit in Richtung Aufnahmekörper 12" verschoben ist dass der als Aktivierungshilfe dienende Ringbund 72 an der freien Stirnseite des Austragstutzens 18'' anliegt und eine Verbindung zwischen dem Aufnahmeraum des Aufnahmekörpers 12" und dem an die Umgebung führenden Axialkanal 42 der Adaptereinrichtung hergestellt ist. In Fig. 10 ist die Vorrichtung 70 im entleerten Zustand dargestellt, in dem die Kolbeneinheit 14" an dem Verschlusszapfen 36" anliegt.

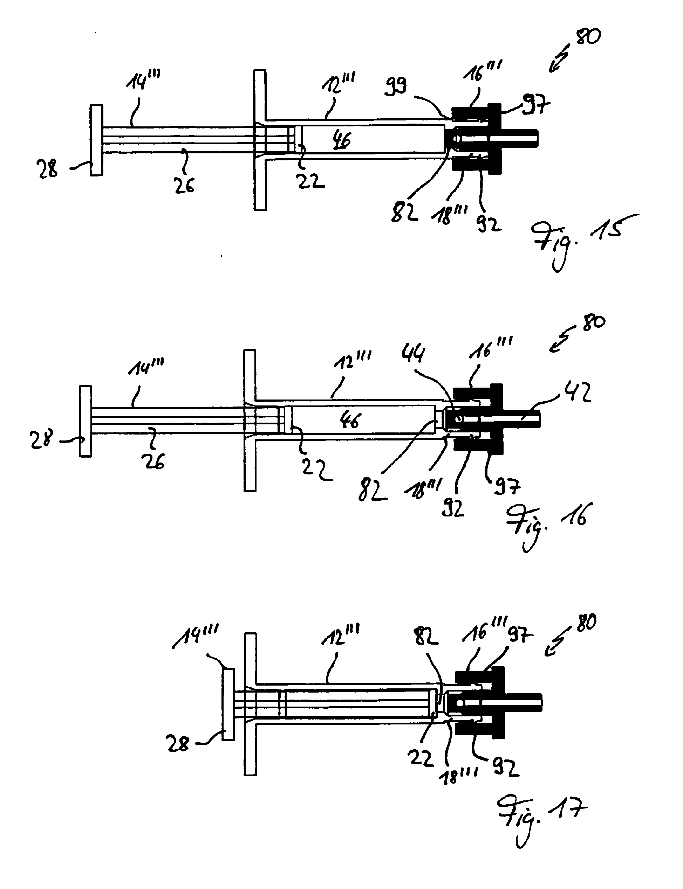

In den Figuren 15 bis 19 ist eine dritte Ausführungsform einer Vorrichtung 80 zum Aufbewahren und Austragen einer fließfähigen Substanz dargestellt. Entsprechend den oben beschriebenen Ausführungsformen umfasst die Vorrichtung 80 im Wesentlichen drei Baueinheiten, nämlich einen im wesentlichen zylindrischen Aufnahmekörper 12''', eine Kolbeneinheit 14''' und eine Applikationseinheit 16 '''.

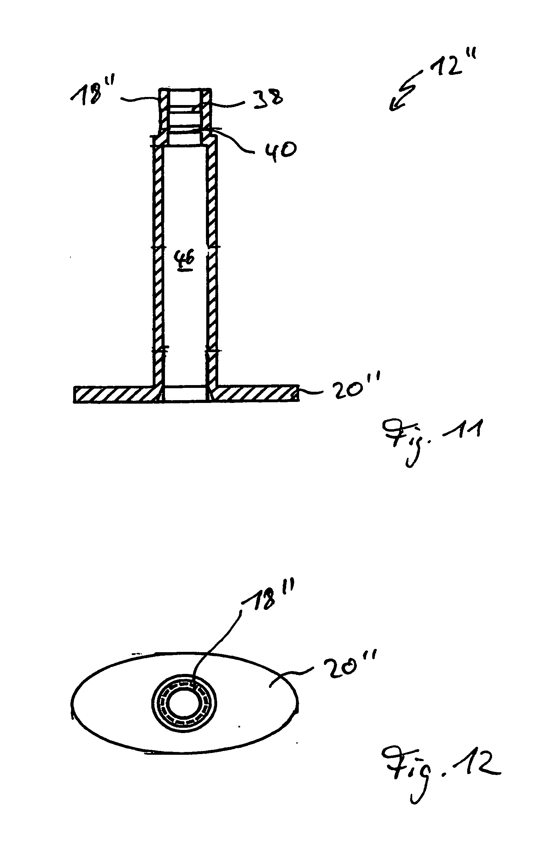

Die Kolbeneinheit 14''' entspricht derjenigen nach den Figuren 8 bis 10. Der zylindrische Aufnahmekörper 12''' unterscheidet sich von demjenigen nach den Figuren 11 und 12 dadurch, dass sich an den Aufnahmeraum 46 eine Auslassöffnung 82 anschließt, die in einen Auslassstutzen 18''' übergeht. An der Innenseite des Auslassstutzens 18''' ist ein Verschlusszapfen 36''' der Applikationseinheit 16''' geführt, der entsprechend der oben beschriebenen Ausführungsformen von einem Querkanal 44 durchsetzt ist, an den sich ein an die Umgebung führender Axialkanal 42 anschließt. Der Verschlusszapfen 36''' umfasst einen Führungsabschnitt 87 vergrößerten Durchmessers und einen Verschlussabschnitt 89 verringerten Durchmessers, welche durch eine Dichtfläche 91 voneinander getrennt sind.

In der in Fig. 15 dargestellten Schließstellung greift der Verschlussabschnitt 89 in die Auslassöffnung 82 des Aufnahmeraums 46 ein und liegt die konische Dichtfläche 91 an einer korrespondierenden Fläche 93 an der Innenseite des Austragstutzens 18''' dichtend an.

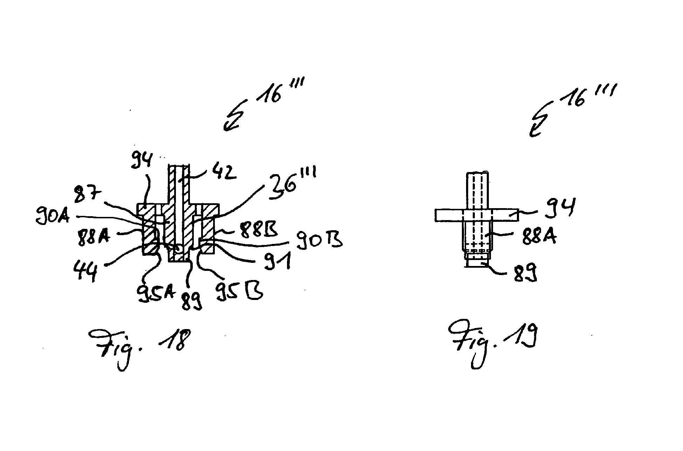

Die Applikationseinheit 16''', die in den Figuren 18 und 19 jeweils in Alleinstellung dargestellt ist, weist des Weiteren eine Grundplatte 94 auf, von der zwei Schenkel 88A und 88B in Richtung des Aufnahmekörpers 12''' parallel zur Achse des Axialkanals 42 vorspringen, die zur Fixierung der Applikationseinheit 16''' an dem Austragstutzen 18''' dienen. Die Schenkel 88A, 88B sind jeweils federelastisch ausgebildet bzw. an der Grundplatte 94 gelagert und mit einer Rastnase 90A bzw. 90B versehen, die mit einem Rastbund 92 am Umfang des Austragstutzens 18''' zusammenwirkt und so die Applikationseinheit 16''' an dem Austragstutzen 18''' sichert.

Um die Applikationseinheit 16''' in einfacher Weise auf den Austragstutzen 18''' aufsetzen bzw. den Verschlusszapfen 36''' in einfacher Weise in den Verschlusszapfen 18''' einführen zu können, weist der Rastbund 92 an seiner dem Aufnahmeraum 46 abgewandten Seite eine Schräge 97 auf, die beim Aufschieben der Applikationseinheit 16''' mit Schrägen 95A und 95B der Schenkel 88A und 88B zusammenwirkt.

In der in Fig. 15 dargestellten Schließstellung greift der Verschlussabschnitt 89 des Verschlusszapfens 36''' in die Auslassöffnung 82 des Aufnahmeraums 46 ein. Die freien Stirnseiten der Schenkel 88A, 88B liegen hierbei an einem einen Anschlag bildenden Ringbund 99 des Aufnahmekörpers 12''' an. Zum Aktivieren der Vorrichtung 80 wird die Applikationseinheit 16''' gegenüber dem Aufnahmekörper 12''' in der der Kolbeneinheit 14''' abgewandten Richtung verschoben, bis die Rastnasen 90A, 90B der Schenkel 88A, 88B an dem Rastbund 92 des Austragstutzens 18''' anschlagen. Der Verschlussabschnitt 89 des Verschlusszapfens 36''' wird damit aus der Auslassöffnung 82 gezogen. Dadurch wird über die Auslassöffnung 82 eine Fluidverbindung zwischen dem Aufnahmeraum 46 des Aufnahmekörpers 12''' und dem Querkanal 44 der Applikationseinheit 16''' hergestellt ist. Durch einen manuellen, axialen Druck auf die Kolbeneinheit 14''' in Richtung der Applikationseinheit 16''' kann dann die in dem Aufnahmeraum 46 enthaltene fleißfähige Substanz aus der Vorrichtung 80 ausgetragen und appliziert werden kann.

## Patentansprüche

1. Vorrichtung zur Aufnahme und zum Austragen einer fließfähigen Substanz, umfassend einen spritzenartigen Aufnahmekörper (12, 12', 12", 12"'), in dem ein Aufnahmeraum (46, 46') ausgebildet und ein Kolben (22) axial verschieblich geführt ist, wobei der Aufnahmekörper (12, 12', 12", 12"') an seiner dem Kolben (22) abgewandten Stirnseite mit einem im Wesentlichen zylindrischen Austragsstutzen (18, 18', 18", 18"') versehen ist, wobei an der Innenseite des Austragsstutzens (18, 18', 18", 18"') ein Verschlusszapfen (36, 36', 36", 36"') geführt ist, der von mindestens einem Querkanal (44, 44') durchsetzt ist, von dem ein Axialkanal (42, 42') abzweigt, wobei in einer Schließstellung des Verschlusszapfens (36, 36', 36", 36"') eine Fließverbindung zwischen dem Querkanal (44, 44') und dem Aufnahmeraum (46, 46') gesperrt ist und in einer Aktivierungsstellung des Verschlusszapfens (36, 36', 36", 36"') eine Fließverbindung zwischen dem Querkanal und dem Aufnahmeraum (46, 46') freigegeben ist, so dass die fließfähige Substanz über den Querkanal (44, 44') und den Axialkanal (42, 42') aus dem Aufnahmeraum (46, 46') austragbar ist, **dadurch gekennzeichnet, dass** an der Innenseite des Austragsstutzens (18, 18', 18") zwei ringförmige Dichtlippen (40) vorgesehen sind, an dem der Verschlusszapfen (36, 36', 36") geführt ist und der Querkanal (44, 44') einen Durchmesser hat, der gleich dem oder kleiner als der Abstand zwischen den beiden Dichtlippen (38, 40) ist.

2. Vorrichtung zur Aufnahme und zum Austragen einer fließfähigen Substanz, umfassend einen spritzenartigen Aufnahmekörper (12"'), in dem ein Aufnahmeraum (46) ausgebildet und ein Kolben (22) axial verschieblich geführt ist, wobei der Aufnahmekörper (12"') an seiner dem Kolben (22) abgewandten Stirnseite mit einem im Wesentlichen zylindrischen Austragsstutzen (18"') versehen ist, wobei an der Innenseite des Austragsstutzens (18"') ein Verschlusszapfen (36"') geführt ist, der von mindestens einem Querkanal (44, 44') durchsetzt ist, von dem ein Axialkanal (42) abzweigt, wobei in einer Schließstellung des Verschlusszapfens (36"') eine Fließverbindung zwischen dem Querkanal (44,) und dem Aufnahmeraum (46) gesperrt ist und in einer Aktivierungsstellung des Verschlusszapfens (36"') eine Fließverbindung zwischen dem Querkanal und dem Aufnahmeraums (46) freigegeben ist, so dass die fließfähige Substanz über den Querkanal (44) und den Axialkanal (42) aus dem Aufnahmeraum (46) austragbar ist, **dadurch gekennzeichnet, dass** der Verschlusszapfen (36"') einen Führungsabschnitt (87) vergrößerten Durchmessers und einen Verschlussabschnitt (89) verringerten Durchmessers aufweist und der Führungsabschnitt (87) und der Verschlussabschnitt (89) durch eine Dichtfläche (91) voneinander getrennt sind.

3. Vorrichtung nach Anspruch 1, **dadurch gekennzeichnet, dass** der Aufnahmekörper (12) einen seitlichen Einführkanal (52) für einen Stoff hat.

4. Vorrichtung nach Anspruch 2, **dadurch gekennzeichnet, dass** der Einführkanal (52) eine einseitige trichterartige Aufweitung einer Wandung des Aufnahmekörpers (52) in dem dem Austragsstutzen (36) abgewandten Endbereich ist.

5. Vorrichtung nach Anspruch 2, **dadurch gekennzeichnet, dass** der Verschlusszapfen (36"') zum Sperren der Fließverbindung zwischen dem Aufnahmeraum (46) und dem Querkanal (44"') in seiner Schließstellung dichtend in eine Auslassöffnung (82) des Aufnahmeraums (46) eingreift.

6. Vorrichtung nach Anspruch 5, **dadurch gekennzeichnet, dass** der Verschlusszapfen (36"') eine konische Dichtfläche (84) hat, die in Schließstellung des Verschlusszapfens (36"') an einer korrespondierenden Fläche (86) des Austragstutzens (18"') anliegt, die sich an die Auslaßöffnung (82) anschließt.

7. Vorrichtung nach einem der Ansprüche 1 bis 6, **dadurch gekennzeichnet, dass** der Verschlusszapfen (36, 36', 36", 36"') Bestandteil einer Applikationseinrichtung (16, 16', 16", 16"') und/oder einer Adaptereinrichtung ist.

8. Vorrichtung nach Anspruch 7, **dadurch gekennzeichnet, dass** die Applikationseinrichtung kanülenartig ausgebildet ist.

9. Vorrichtung nach Anspruch 7, **dadurch gekennzeichnet, dass** die Applikationseinrichtung (16) mit einer Düse (32) versehen ist.

10. Vorrichtung nach einem der Ansprüche 1 bis 9, **dadurch gekennzeichnet, dass** der Verschlusszapfen (36') mittels einer Feder (60) gelagert und in Schließstellung vorgespannt ist.

11. Vorrichtung nach Anspruch 10, **dadurch gekennzeichnet, dass** die Feder (60) gekapselt ist.

12. Vorrichtung nach Anspruch 10 oder 11, **dadurch gekennzeichnet, dass** die Feder (60) den Austragsstutzen (18') umgreift.

13. Vorrichtung nach einem der Ansprüche 1 bis 12, **dadurch gekennzeichnet, dass** der Verschlusszapfen (36"') an dem Austragsstutzen (18"') verrastet ist.

14. Vorrichtung nach Anspruch 13, **dadurch gekennzeichnet, dass** eine Applikationseinrichtung (16"') oder Adaptereinrichtung, an der der Verschlusszapfen (36"') ausgebildet ist, mindestens zwei Schenkel (88A 88B) aufweist, die von einer Grundplatte (94) in Richtung des Aufnahmeraums (46) im Wesentlichen parallel zu der Achse des Axialkanals (42) vorspringen und an deren jeweils eine Rastnase (90A, 90B) ausgebildet ist, die mit einem korrespondierenden Rastbund (92) am Umfang des Austragstutzens (18"') zusammenwirkt.

15. Vorrichtung nach einem der Ansprüche 1 bis 14, **dadurch gekennzeichnet, dass** der Verschlusszapfen mittels Fixiereinrichtungen in Schließstellung und/oder in Aktivierungsstellung sicherbar ist.

## Claims

1. Device for retaining and dispensing a free-flowing substance, including a syringe-like retaining body (12, 12', 12", 12"') in which a retaining space (46, 46') is formed and in which a piston (22) is axially displaceably guided, wherein on its front end facing away from the piston (22) the retaining body (12, 12', 12", 12"') is equipped with an essentially cylindrical dispensing nozzle (18, 18', 18", 18"'), wherein a locking pin (36, 36', 36", 36"') through which at least one transverse channel (44, 44') passes, with an axial channel (42, 42') branching off therefrom, is guided on the inside of the dispensing nozzle (18, 18', 18", 18"'), wherein in a closed position of the locking pin (36, 36', 36", 36"') a flow connection between the transverse channel (44, 44') and the retaining space (46, 46') is blocked and in an activation position of the locking pin (36, 36', 36", 36"') a flow connection between the transverse channel and the retaining space (46, 46') is released, so that the free-flowing substance can be dispensed from the retaining space (46, 46') via the transverse channel (44, 44') and the axial channel (42, 42'),
**characterized in that**
on the inside of the dispensing nozzle (18, 18', 18") two ring-shaped sealing lips (38, 40) are provided along which the locking pin (36, 36', 36") is guided, and the transverse channel (44, 44') has a diameter which is equal to or less than the distance between the two sealing lips (38, 40).

2. Device for retaining and dispensing a free-flowing substance, including a syringe-like retaining body (12"') in which a retaining space (46) is formed and in which a piston (22) is axially displaceably guided, wherein on its front end facing away from the piston (22) the retaining body (12"') is equipped with an essentially cylindrical dispensing nozzle (18"'), wherein a locking pin (36"') through which at least one transverse channel (44, 44') passes, with an axial channel (42) branching off therefrom, is guided on the inside of the dispensing nozzle (18"'), wherein in a closed position of the locking pin (36"') a flow connection between the transverse channel (44) and the retaining space (46) is blocked and in an activation position of the locking pin (36"') a flow connection between the transverse channel and the retaining space (36) is released, so that the free-flowing substance can be dispensed from the retaining space (46) via the transverse channel (44) and the axial channel (42),
**characterized in that**
the locking pin (36"') comprises a guide section (87) of an enlarged diameter and a sealing section (89) of a reduced diameter, the guide section (87) and the sealing section (89) being separated from one another by means of a sealing face (91).

3. Device according to Claim 1,
**characterized in that**
the retaining body (12) features a lateral insertion channel (52) for a substance.

4. Device according to Claim 2,
**characterized in that**
the insertion channel (52) has a single-sided funnel-shaped widening of a wall of the retaining body (52) in the end portion facing away from the dispensing nozzle (36).

5. Device according to Claim 2,
**characterized in that**
the locking pin (36"') for blocking the flow connection between the retaining space (46) and the transverse channel (44"') in its closed position sealingly engages in an outlet opening (82) of the retaining space (46).

6. Device according to Claim 5,
**characterized in that**
the locking pin (36') comprises a conical sealing face (84) which in the closed position of the locking pin (36"') rests against a corresponding face (86) of the dispensing nozzle (18"') that is connected to the outlet opening (82).

7. Device according to any of Claims 1 to 6,
**characterized in that**
the locking pin (36, 36', 36", 36"') is part of an application means (16, 16', 16", 16"') and/or an adapter means.

8. Device according to Claim 7,
**characterized in that**
the application means is designed like a cannula.

9. Device according to Claim 7,
**characterized in that**
the application means (16) is equipped with a nozzle (32).

10. Device according to any of Claims 1 to 9,
**characterized in that**
the locking pin (36') is supported by means of a spring (60) and is prestressed in direction of the closed position.

11. Device according to Claim 10,
**characterized in that**
the spring (60) is encapsulated.

12. Device according to Claim 10 or 11,
**characterized in that**
the spring (60) surrounds the dispensing nozzle (18').

13. Device according to any of Claims 1 to 12,
**characterized in that**
the locking pin (36"') is locked on the dispensing nozzle (18"').

14. Device according to Claim 13,
**characterized in that**
an application means (16"') or an adapter means on which the locking pin (36"') is formed comprises at least two legs (88A, 88B) which protrude from a base plate (94) in the direction of the retaining space (46) essentially parallel to the axis of the axial channel (42) and on each of which a catch nose (90A, 90B) is formed, interacting with a corresponding catch collar (92) at the circumference of the dispensing nozzle (18"').

15. Device according to any of Claims 1 to 14,
**characterized in that**
the locking pin can be secured by means of fixing devices in the closed position and/or in the activation position.

## Revendications

1. Dispositif destiné à la réception et la distribution d'une substance coulante, comprenant un corps récepteur (12, 12', 12", 12"') de type seringue, en dedans duquel un espace récepteur (46, 46') est formé et un piston (22) est guidé de manière axialement déplaçable, dans lequel le corps récepteur (12, 12', 12", 12"') sur son côté frontal détourné du piston (22) est muni d'une buse distributrice (18, 18', 18", 18"') de forme essentiellement cylindrique, dans lequel une broche d'enclenchement (36, 36', 36", 36"'), à travers laquelle passe au moins un canal transversal (44, 44') avec un canal axial (42, 42') bifurquant à partir de celui-ci, est guidée sur le côté intérieur de la buse distributrice (18, 18', 18", 18"'), dans lequel une connexion d'écoulement entre le canal transversal (44, 44') et l'espace récepteur (46, 46') est bloquée dans une positon fermée de la broche d'enclenchement (36, 36', 36", 36"') et une connexion d'écoulement entre le canal transveral et l'espace récepteur (46, 46') est libérée dans une position d'activation de la broche d'enclenchement (36, 36', 36", 36"') de sorte que la substance coulante peut être distribuée à partir de l'espace récepteur (46, 46') via le canal transversal (44, 44') et le canal axial (42, 42'),
**caractérisé en ce que**
sur le coté intérieur de la buse distributrice (18, 18', 18") deux lèvres d'étanchéité (38, 40) de forme annulaire sont prévus, la broche d'enclenchement (36, 36', 36") étant guidée le long de celles-ci, et le canal transversal présente un diamètre égal à ou inférieur à la distance entre les deux lèvres d'étanchéité (38, 40).

2. Dispositif destiné à la réception et à la distribution d'une substance coulante, comprenant un corps récepteur (12"') de type seringue, en dedans duquel un espace récepteur (46) est formé et un piston (22) est guidé de manière axialement déplaçable, dans lequel le corps récepteur (12"') sur son côté frontal détourné du piston (22) est muni d'une buse distributrice (18"') de forme essentiellement cylindrique, dans lequel une broche d'enclenchement (36"'), à travers laquelle passe au moins un canal transverse (44, 44') avec un canal axial (42) bifurquant à partir de celui-ci, est guidée sur le côté intérieur de la buse distributrice (18"'), dans lequel une connexion d'écoulement entre le canal transversal (44) et l'espace récepteur (46) est bloquée dans une position fermée de la broche d'enclenchement (36"') et une connexion d'écoulement entre le canal transversal et l'espace récepteur (36) est libérée dans une position d'activation de la broche d'enclenchement (36"'), de sorte que la substance coulante peut être distribuée à partir de l'espace récepteur (46) via le canal transversal (44) et la canal axial (42),
**caractérisé en ce que**
la broche d'enclenchement (36"') présente une section de guidage (87) d'un diamètre élargi et une section d'étanchéité (89) d'un diamètre réduit, la section de guidage (87) et la section d'étanchéité (89) étant séparées l'une de l'autre au moyen d'une face d'étanchéité.

3. Dispositif selon la revendication 1,
**caractérisé en ce que**
le corps récepteur (12) présente un canal d'insertion (52) lateral pour une substance.

4. Dispositif selon la revendication 2,
**caractérisé en ce que**
le canal d'insertion (52) présente un élargissement à un seul côté en forme d'entonnoir d'une paroi du corps récepteur (52) dans la section terminale détournée de la buse distributrice (36).

5. Dispositif selon la revendication 2,
**caractérisé en ce que**
la broche d'enclenchement (36"') pour le blocage de la connexion d'écoulement entre l'espace récepteur (46) et le canal transverse (44"') dans sa position fermée s'engrène de manière étanche dans une ouverture de sortie (82) de l'espace récepteur (46).

6. Dispositif selon la revendication 5,
**caractérisé en ce que**
la broche d'enclenchement (36') présente une face d'étanchéité (84) conique qui, dans la position fermée de la broche d'enclenchement (36"'), vient en butée contre une face (86) correspondante de la buse distributrice (18"') qui est reliée à l'ouverture de sortie (82).

7. Dispositif selon l'une quelconque des revendications 1 à 6,
**caractérisé en ce que**
la broche d'enclenchement (36, 36', 36", 36"') est partie d'un dispositif d'application (16, 16', 16", 16"') et/ou d'un dispositif adaptateur.

8. Dispositif selon le revendication 7,
**caractérisé en ce que**
le dispositif d'application est conçu en forme de cannule.

9. Dispositif selon la revendication 7,
**caractérisé en ce que**
le dispositif d'application (16) est muni d'une tuyère (32).

10. Dispositif selon l'une quelconque des revendications 1 à 9,
**caractérisé en ce que**
la broche d'enclenchement (36') est appuyée au moyen d'un ressort (60) et est précontrainte dans la position fermée.

11. Dispositif selon la revendication 10,
**caractérisé en ce que**
le ressort (60) est encapsulé.

12. Dispositif selon la revendication 10 ou 11,
**caractérisé en ce que**
le ressort (60) entoure la buse distributrice (18').

13. Dispositif selon l'une quelconque des revendications 1 à 12,
**caractérisé en ce que**
la broche d'enclenchement (36"') est enclenchée dans la buse distributrice (18"').

14. Dispositif selon la revendication 13,
**caractérisé en ce qu'**
un dispositif d'application (16"') ou un dispositif d'adaptateur, sur lequel la broche d'enclenchement (36"') est formée, présente au moins deux branches (88A, 88B) faisant saillie à partir d'une plaque de base (94) en direction de l'espace récepteur (46) essentiellement en parallèle à l'axe du canal axial (42), et présentant chacune un nez d'enclenchement (90A, 90B) interagissant avec un collet d'enclenchement (92) correspondant sur la circonférence de la buse distributrice (18"').

15. Dispositif selon l'une quelconque des revendications 1 à 14,
**caractérisé en ce que**
la broche d'enclenchement peut être sécurisée dans la position fermée et/ou dans la position d'activation au moyen de dispositifs de fixation.
